# EUROPEAN PATENT SPECIFICATION

(11) **EP 0 586 336 B1**
(45) Date of publication and mention of the grant of the patent: **26.03.1997**
(21) Application number: 93810569.9
(22) Date of filing: 10.08.1993
(51) Int. Cl.: C08G 59/50, C07C 317/36

(54) **2,2'-Dimethyl-5,5'-diaminodiphenyl sulfone**
2,2'-Dimethyl-5,5'-Diaminodiphenyl Sulphon
2,2'-Diméthyl-5,5'-diaminodiphenyl sulfone

(30) Priority: 18.08.1992 US 931754
(43) Date of publication of application: 09.03.1994
(73) Proprietor: Ciba Specialty Chemicals Holding Inc., 4057 Basel (CH)
(72) Inventor: Lee, Byung, Easton, CT 06612 (US); King, John, Ridgefield, CT 06877 (US); Fu, Bernie, Stamford, CT 06905 (US)

(56) References cited:
- EP-A- 0 193 082
- EP-A- 0 335 706
- FR-A- 2 519 639
- GB-A- 798 177

## Description

The invention relates to 2,2'-Dimethyl-5,5'-diaminodiphenyl sulfone and a resin system comprising the said sulfone as a hardener.

US-Patent No. 4,500,582 discloses a resinous hardener system comprising an adduct of an epoxy resin with a diamino diphenylsulfones of the formula 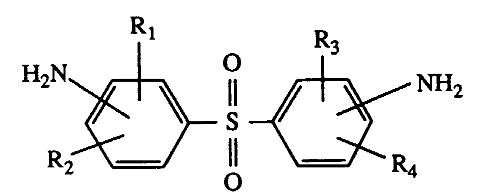 wherein R₁, R₂, R₃ and R₄ are independently hydrogen, straight and branched chain alkyl of 1 to 12 carbon atoms, preferably 1 to 4 carbon atoms or halogen.

It is the primary object of this invention to provide a modified hardener system for epoxy resins.

It is a further object to provide such a hardener system which improves upon the performance of diamino diphenylsulfone hardeners.

It is another object to utilize such hardener systems with a wide variety of epoxy resins to provide prepreg or laminating resins of improved performance characteristics.

Various other objects and advantages of this invention will become apparent from the following descriptive material and illustrative examples.

Applicants have surprisingly found that resin systems comprising epoxy resins and 2,2'-dimethyl-5,5'-diamino diphenyl sulfone, as a hardener, exhibits improved processing characteristics.

2,2'-dimethyl-5,5'-diamino diphenyl sulfone is prepared from 2,2'-dimethyl-5,5'-dinitrodiphenyl sulfone by conventional reduction.

The hardener of the present invention lends itself for use in primary composite structures. The resin system of the present invention is useful in a wide area of applications such as coatings, molded castings, adhesives and filament wound structures.

Resin systems according to the present invention comprise an epoxy resin and said sulfone. The sulfone is present in an amount ranging from about 0.5 to about 1.0, preferably about 0.8 to about 1.0 equivalents of aminohydrogen per equivalent of epoxy.

Suitable epoxy resins include epoxide resins based on polyhydric phenols such as those based on bisphenol A, F, and S, epoxidation products of cresol novolacs, and epoxidation products of phenol novolacs; hydantoin epoxide resins; polyglycidyl esters; glycidylated aromatic amines; glycidylated aminophenols; and certain cycloaliphatic epoxy resins. Tetraglycidylated methylene dianiline is preferred for purposes of the instant invention. In addition, in adhesive, coating and filament winding applications, resin based on the diglycidyl ether of bisphenol A is widely used.

Techniques for preparing prepregs are well known to those skilled in the art. In terms of honeycomb skins, graphite, glass, Kevlar reinforced skins as well as others, can be readily prepared from the instant systems. Correspondingly, techniques for preparing laminates are well known. Such laminates may be prepared by compression or autoclave molding and may comprise a broad range of thicknesses.

Apart from the above areas of utility, the sulfone of this invention is useful as a curing agent for a wide variety of epoxy resins in various heat cured applications. When combined with di- and polyepoxides, at generally stoichiometric amounts, and cured at elevated temperatures, a network of high crosslink density occurs. Accordingly, the expression "cure" as used herein, denotes the conversion of the above sulfone and epoxide material into insoluble and infusible crosslinked products, with simultaneous shaping to give shaped articles such as castings, pressings or laminates, or to give two-dimensional structures such as coatings, enamels or adhesive bonds. The sulfone of the present invention is particularly advantageous for the formation of coatings because of the improved compatibility with resins and the improved toughness of the resulting cured coatings.

The compositions of the present invention can furthermore be mixed, at any stage before cure, with usual modifiers such as extenders, fillers and reinforcing agents, pigments, dyestuffs, organic solvents, plasticizers, tackifiers, rubbers, accelerators, diluents, and the like. As extenders, reinforcing agents, fillers and pigments which can be employed in the curable mixtures according to the invention there may be mentioned, for example: coal tar, bitumen, glass fibers, boron fibers, carbon fibers, cellulose, polyethylene powder, polypropylene powder, mica, asbestos, quartz powder, gypsum, antimony trioxide, bentones, silica aerogel ("Aerosil"), lithopone, barite, titanium dioxide, carbon black, graphite, iron oxide, or metal powders such as aluminum powder or iron powder. It is also possible to add other usual additives, for example, flameproofing agents, agents for conferring thixotropy, flow control agents such as silicones, cellulose acetate butyrate, polyvinyl butyral, waxes, stearates and the like (which are in part also used as mold release agents) to the curable mixtures.

It is also possible in adhesive formulations, for example, to add rubbers such as carbonyl-terminated acrylonitrile-butadiene rubber, modifying resins such as triglycidyl p-aminophenol, accelerators such as boron trifluoride monoethylamine complexes or imidazole complexes, and other additional hardeners such as dicyandiamide.

The curable mixtures can be manufactured in the usual manner with the aid of known mixing equipment (stirrers, kneaders, rollers and the like).

The following examples will further illustrate the embodiments of the instant invention, but are not intended to limit this invention.

### Examples 1-3

These examples illustrate the preparation of a curable mixture according to the present invention and the preparation of 2 controls for comparative purposes. The curable mixtures are prepared by heating the resin in a 3 neck flask to a temperature just sufficient to dissolve the hardener in an oil bath. The hardener is then added slowly and the mixture stirred until all solids are dissolved. The resulting prepolymer is then degassed with a mechanical vacuum pump and the prepolymer is then poured into a preheated aluminum mold and cured.

A du Pont 9900 Differential Scanning Colorimeter is used to determine reaction temperatures; gel times are determined using a hot plate, system viscosities and pot lives are measuring with a Brookfield viscometer, viscosity isotherms are obtained with a Rheometrics Dynamic Spectrometer (RDA 700) and Dynamic Young's Moduli are measured by Dynamic Mechanical Analysis (du Pont DMA 983). The formulations are presented in Table 1 and the physical properties are presented in Table 2.

**Table 1**

| Formulations | | | |
|---|---|---|---|
| | (Parts by weight) | | |
| Component | Ex. 1 | Ex. 2 | Ex. 3 |
| MY 720 (tetraglycidylated methylene dianiline from CIBA-GEIGY Corp.) | 100 | 100 | 100 |
| HT 976 (4,4'-diamino diphenyl sulfone from CIBA-GEIGY Corp.) | - | 44 | - |
| 2,2'-dimethyl-5,5'-diamino diphenyl sulfone | 50 | - | - |
| 3,3'-diamino diphenyl sulfone | - | - | 44 |

The following Table 2 shows that the DSC identified exotherm initiation (Ti) and peak (Tpk) temperatures for Example 1 in accordance with the present invention are 20°C and 40°C lower, respectively, than those of control Example 2. At 100°C, the viscosity of Example 1 in accordance with the present invention is almost twice that of both control Examples 2 and 3. Furthermore, the pot-life (time required to double the initial viscosity) at 100°C of Example 1 is significantly shorter (2.0 hours) than that of control Example 2 (> 2.5 hours) and control Example 3 (2.5 hours). The resin system of Example 1 gelled within 5 minutes at 177°C, whereas under the same conditions the resin systems of control Examples 2 and 3 gelled in 17.9 and 7.7 minutes, respectively.

**Table 2**

| Physical Properties | | | |
|---|---|---|---|
| DSC | Ex. 1 | Ex. 2 | Ex. 3 |
| Ti, °C | 150 | 170 | 135 |
| Tpk, °C | 207 | 245 | 223 |
| ΔH, J/g | 557 | 557 | 322 |

| VISCOSITY | | | |
|---|---|---|---|
| at 100°C (cps) | 1080 | 580 | 600 |

| POT-LIFE (Time to double initial viscosity). | | | |
|---|---|---|---|
| at 100°C (hrs) | 2.0 | >2.5 | 2.5 |

| GEL TIME AT 177°C | | | |
|---|---|---|---|
| (min.) | 4.8 | 17.9 | 7.7 |

### Examples 4 and 5

These examples illustrate the preparation of a curable mixture according to the present invention and the preparation of a control for comparative purposes. The formulations are provided in Table 3 and the physical properties are presented in Table 4.

**Table 3**

| Formulations | | |
|---|---|---|
| Component | (parts by weight) | |
| | Ex. 4 | Ex. 5 |
| RD 87-160 (Tetra-functional Epoxy from CIBA-GEIGY Corp.) | 100 | 100 |
| HT 976 (4,4'-diamino diphenyl sulfone from CIBA-GEIGY Corp.) | - | 44 |
| 2,2'-dimethyl-5,5'-diaminodiphenyl sulfone | 50 | - |

**Table 4**

| Physical Properties | | | |
|---|---|---|---|
| DSC | | Ex. 4 | Ex. 5 |
| Ti, °C | | 165 | 170 |
| Tpk, °C | | 228 | 254 |
| ΔH, J/g | | 432 | 439 |

| VISCOSITY | | | |
|---|---|---|---|
| at 100°C (cps) | | 265 | |

| GEL TIME AT 177°C | | | |
|---|---|---|---|
| (min.) | | 11.1 | 37.2 |

| CURE SCHEDULE | | | |
|---|---|---|---|
| Room temperature to 177°C using an increase in temperature of 2°C/min, then 4 hours at 177°C | | | |

| DMA MODULUS, DRY | | | |
|---|---|---|---|
| (KSI) | 25°C | 548 | 577 |
| | 150°C | 430 (79%) | 438 (76%) |
| | 200°C | 333 (61%) | 340 (59%) |
| | Tg, °C | 220 | 220 |

Values in parentheses indicate retention of room temperature modulus.

The DSC identified initiation temperature of the resin system of Example 4 is equivalent to control Example 5. However, the peak (Tpk) temperature of the resin system of Example 4 is approximately 30°C lower than that of the control Example 5. At 177°C the resin system of Example 4 gelled three times faster than that of control Example 5 (11.1 minutes as compared with 37.2 minutes, respectively).

Thus, the results establish that 2,2'-dimethyl-5,5'-diaminodiphenyl sulfone exhibits a higher melting point, reactivity, and equivalent weight than 3,3'-diaminodiphenyl sulfone and 4,4'-diaminodiphenyl sulfone. The resin system in accordance with the present invention exhibits good thermal-mechanical properties.

## Claims

1. 2,2'-Dimethyl-5,5'-diaminodiphenyl sulfone.

2. A resin system comprising an epoxy resin and 2,2'-dimethyl-5,5'-diaminodiphenyl sulfone.

3. A resin system according to claim 2 wherein said 2,2'-dimethyl-5,5'-diaminodiphenyl sulfone is present in an amount ranging from about 0.5 to about 1.0 equivalents of aminohydrogen per equivalent of epoxy.

4. A resin system according to claim 2 wherein said 2,2'-dimethyl-5,5'-diaminodiphenyl sulfone is present in an amount ranging from about 0.8 to about 1.0 equivalents of aminohydrogen per equivalent of epoxy.

5. A resin system acccording to claim 2 wherein said epoxy resin is selected from the group consisting of epoxide resins based on polyhydric phenols, epoxidation products of cresol novolacs and phenol novolacs, hydantoin epoxide resins, polyglycidyl esters, glycidylated aromatic amines, glycidylated aminophenols, and cycloaliphatic epoxy resins.

6. A resin system according to claim 2 wherein said epoxy resin is tetraglycidylated methylene dianiline.

7. A resin system according to claim 2 wherein said epoxy resin is based on the diglycidyl ether of bisphenol A.

8. A resin system according to claim 2 further comprising modifiers, fillers and reinforcing agents, pigments, dyestuffs, organic solvents, plasticizers, tackifiers, rubbers, accelerators, diluents or combinations thereof.

9. A resin system according to claim 2 further comprising coal tar, bitumen, glass fibers, boron fibers, carbon fibers, cellulose, polyethylene powder, polypropylene powder, mica, asbestos, quartz powder, gypsum, antimony trioxide, bentones, silica aerogel, lithopone, barite, titanium dioxide, carbon black, graphite, iron oxide, metal powders or a combination thereof.

10. A resin system according to claim 2 further comprising flameproofing agents, agents for conferring thixotropy, flow control agents, cellulose acetate butyrate, polyvinyl butyral, waxes, stearates or a combination thereof.

11. A resin system according to claim 2 further comprising rubbers, modifying resins accelerators, additional hardeners and combinations thereof.

12. A resin system according to claim 2 further comprising carbonyl-terminated acrylonitrile-butadiene rubber, triglycidyl p-aminophenol, boron trifluoride monoethylamine complexes or imidazole complexes, dicyandiamide and combinations thereof.

## Patentansprüche

1. 2,2'-Dimethyl-5,5'-diaminodiphenylsulfon.

2. Harzsystem, umfassend ein Epoxidharz und 2,2'-Dimethyl-5,5'-diaminodiphenylsulfon.

3. Harzsystem nach Anspruch 2, wobei das 2,2'-Dimethyl-5,5'-diaminodiphenylsulfon in einer Menge im Bereich von etwa 0,5 bis etwa 1,0 Äquivalenten Aminowasserstoff pro Äquivalent Epoxy vorliegt.

4. Harzsystem nach Anspruch 2, wobei das 2,2'-Dimethyl-5,5'-diaminodiphenylsulfon in einer Menge im Bereich von etwa 0,8 bis etwa 1,0 Äquivalenten Aminowasserstoff pro Äquivalent Epoxy vorliegt.

5. Harzsystem nach Anspruch 2, wobei das Epoxidharz ausgewählt ist aus der Gruppe, bestehend aus Epoxidharzen, basierend auf mehrwertigen Phenolen, Epoxidierungsprodukten von Cresolnovolaken und Phenolnovolaken, Hydantoinepoxidharzen, Polyglycidylestern, glycidylierten aromatischen Aminen, glycidylierten Aminophenolen und cycloaliphatischen Epoxidharzen.

6. Harzsystem nach Anspruch 2, wobei das Epoxidharz tetraglycidyliertes Methylendianilin darstellt.

7. Harzsystem nach Anspruch 2, wobei das Epoxidharz auf dem Diglycidylether von Bisphenol A basiert.

8. Harzsystem nach Anspruch 2, weiter umfassend Modifizierungsmittel, Füllstoffe und Verstärkungsmittel, Pigmente, Farbstoffe, organische Lösungsmittel, Weichmacher, Haftmittel, Kautschuke, Beschleuniger, Verdünnungsmittel oder Kombinationen davon.

9. Harzsystem nach Anspruch 2, weiter umfassend Kohlenteer, Bitumen, Glasfasern, Borfasern, Kohlefasern, Cellulose, Polyethylenpulver, Polypropylenpulver, Glimmer, Asbest, Quarzpulver, Gips, Antimontrioxid, Bentonit, Siliciumdioxidaerogel, Lithopone, Baryt, Titandioxid, Ruß, Graphit, Eisenoxid, Metallpulver oder eine Kombination davon.

10. Harzsystem nach Anspruch 2, weiter umfassend Flammschutzmittel, Mittel zum Verleihen von Thixotropie, Fließregelungsmittel, Celluloseacetatbutyrat, Polyvinylbutyral, Wachse, Stearate oder eine Kombination davon.

11. Harzsystem nach Anspruch 2, weiter umfassend Kautschuke, modifizierende Harze, Beschleuniger, weitere Härter und Kombinationen davon.

12. Harzsystem nach Anspruch 2, weiter umfassend Acrylnitril-Butadien-Kautschuk mit endständigen Carbonylgruppen, Triglycidyl-p-aminophenol, Bortrifluorid-Monoethylamin-Komplexe oder -Imidazolkomplexe, Dicyandiamid und Kombinationen davon.

## Revendications

1. 2,2'-diméthyl-5,5'-diaminodiphénylsulfone.

2. Un système résineux comprenant une résine époxy et de la 2,2'-diméthyl-5,5'-diaminodiphénylsulfone.

3. Un système résineux selon la revendication 2, dans lequel ladite 2,2'-diméthyl-5,5'-diaminodiphénylsulfone est présente en une quantité comprise entre environ 0,5 et environ 1,0 équivalent d'hydrogène d'amine par équivalent d'époxy.

4. Un système résineux selon la revendication 2, dans lequel ladite 2,2'-diméthyl-5,5'-diaminodiphénylsulfone est présente en une quantité comprise entre environ 0,8 et environ 1,0 équivalent d'hydrogène d'amine par équivalent d'époxy.

5. Un système résineux selon la revendication 2, dans lequel ladite résine époxy est choisie dans le groupe formé par les résines époxy à base de polyphénols, les produits d'époxydation de novolaques de crésol et de novolaques de phénol, les résines époxy d'hydantoïne, les esters polyglycidyliques, les amines aromatiques glycidylées, les aminophénols glycidylés et les résines époxy cycloaliphatiques.

6. Un système résineux selon la revendication 2, dans lequel ladite résine époxy est la méthylène-dianiline tétraglycidylée.

7. Un système résineux selon la revendication 2, dans lequel ladite résine époxy est à base de l'éther diglycidylique de bisphénol A.

8. Un système résineux selon la revendication 2, comprenant de plus des modificateurs, des charges et agents de renforcement, des pigments, des colorants, des solvants organiques, des plastifiants, des agents d'adhésivité, des caoutchoucs, des accélérateurs, des diluants ou une association d'entre eux.

9. Un système résineux selon la revendication 2, comprenant de plus du goudron de houille, du bitume, des fibres de verre, des fibres de bore, des fibres de carbone, de la cellulose, de la poudre de polyéthylène, de la poudre de polypropylène, du mica, de l'amiante, de la poudre de quartz, du gypse, de l'anhydride antimonieux, des bentones, un aérogel de silice, du lithopone, de la barytine, du bioxyde de titane, du noir de carbone, du graphite, de l'oxyde de fer, des poudres métalliques ou une association d'entre eux.

10. Un système résineux selon la revendication 2, comprenant de plus des agents ignifuges, des agents donnant de la thixotropie, des agents de réglage d'écoulement, de l'acétobutyrate de cellulose, du polyvinylbutyral, des cires, des stéarates ou une association d'entre eux.

11. Un système résineux selon la revendication 2, comprenant de plus des caoutchoucs, des résines modificatrices, des accélérateurs, d'autres durcisseurs, ou une association d'entre eux.

12. Un système résineux selon la revendication 2, comprenant de plus un caoutchouc acrylonitrile-butadiène à terminaison carbonyle, du triglycidyl-*p*-aminophénol, des complexes trifluorure de bore-monoéthylamine ou des complexes d'imidazole, du dicyandiamide ou une association d'entre eux.
